# EUROPEAN PATENT SPECIFICATION

(11) **EP 1 840 222 B1**
(45) Date of publication and mention of the grant of the patent: **23.02.2011**
(21) Application number: 05824023.5
(22) Date of filing: 28.12.2005
(51) Int. Cl.: C12Q 1/68

(54) **THE OPTIMUM METHOD OF AMPLIFICATION ON POLYMERASE CHAIN REACTION**
OPTIMALES VERFAHREN ZUR AMPLIFIKATION NACH POLYMERASEKETTENREAKTION
PROCEDE D'AMPLIFICATION OPTIMALE SUR UNE REACTION EN CHAINE PAR POLYMERASE

(30) Priority: 29.12.2004 CN 200410099186; 03.02.2005 CN 200510023780
(43) Date of publication of application: 03.10.2007
(73) Proprietor: Suzhou Yangtze Delta Academy of Bio-x Science Ltd., Suzhou, Jiangsu 215128 (CN)
(72) Inventor: LI, Haikuo, Shanghai 200030 (CN); HUANG, Jiehuan, Shanghai 200041 (CN); LV, Junhong, Jiading District, Shanghai 201800 (CN); ZHANG, Xiaodong, Shanghai 200233 (CN); HU, Jun, Jiading District, Shanghai 201800 (CN); ZHANG, Zhizhou, Tianjin 300457 (CN); FAN, Chunbai, Jiading District, Shanghai 201800 (CN)
(74) Representative: Walker, Ross Thomson
(86) International application number: PCT/CN2005/002345
(87) International publication number: WO 2006/069537

(56) References cited:
- WO-A-03/048769
- WO-A1-03/095478
- WO-A2-03/033735
- CN-A- 1 381 589
- CN-A- 1 390 955
- CN-A- 1 398 987
- LI MIN ET AL: "Enhancing the efficiency of a PCR using gold nanoparticles" NUCLEIC ACIDS RESEARCH, OXFORD UNIVERSITY PRESS, SURREY, GB, vol. 33, no. 21, 27 November 2005 (2005-11-27), pages E-1841, XP008090941 ISSN: 0305-1048
- ZHU Q. ET AL.: 'Detection of known mutation in hypertrophic cardiomyopathy using oligonucleotide microarrays with nanogold particles/silver stained targets' ACTA ACADEMIAE MEDICINAE MILITARIS TERTIAE vol. 26, no. 19, October 2004, pages 1729 - 1731
- GU Y. AND HE N.: 'Applications of Au Nanoparticles Labeling Method in DNA-detection and the Technology of Genechip' JOURNAL OF CHINA PHARMACEUTICAL UNIVERSITY vol. 34, no. 2, 2003, pages 184 - 189, XP001538832
- HE W. ET AL.: 'Study of PCR Biochip Microprocess Technology' INSTRUMENT TECHNOLOGY AND SENSOR 2003, pages 10 - 13
- MORENO M ET AL: "Selection of aptamers against KMP-11 using colloidal gold during the SELEX process" BIOCHEMICAL AND BIOPHYSICAL RESEARCH COMMUNICATIONS, ACADEMIC PRESS INC. ORLANDO, FL, US, vol. 308, no. 2, 22 August 2003 (2003-08-22), pages 214-218, XP004442963 ISSN: 0006-291X
- HEAP DAVID M ET AL: "PCR amplification using electrolytic resistance for heating and temperature monitoring" BIOTECHNIQUES, vol. 29, no. 5, November 2000 (2000-11), pages 1006-1012, XP008117869 ISSN: 0736-6205

## Description

### FIELD OF THE INVENTION

The present invention relates to a method in biotechnology, particularly an optimized method of polymerase chain reaction amplification.

### BACKGROUND ART

Polymerase Chain Reaction (PCR) is a quick and easy method for generating a large amount of copies of any target DNA sequence in vitro, which is invented by K. Mullis in 1985. Although PCR has been developed to be a mature technique, there are still some problems such as insufficient specificity, sensitivity, and the rate of reaction, needed to be improved in the practical operation. The most remarkable problem is non-specificity existed in different degrees in PCR amplification. Due to the complicated mechanism of PCR, some unavoidable interference side reaction will be happened, for example, the mispairing between primers and templates, the formation of dipolymers by the polymerization of primers, etc., which result lower amplification specificity and lower efficiency of amplification, especially in the selective amplification of specific low copy number DNA, the amplification of single molecule DNA, the amplification of overly long DNA fragments, multiple PCR amplification and so on. Even worse, such interference effects could cause the failure of the amplification reaction. The improvement of the specificity of PCR amplification is not only determined by the optimization of the primers' sequence design, but also is depended on the optimization of the reaction system and procedure. As many efforts have been put into the optimization of the components of PCR system in the past decades, for example, it is proven that the addition of some additives, such as formamide, glycerin, DMSO (dimethyl sulfoxide) into the reaction system, will reduce the non-specific amplification. Unfortunately, the effects of some additives above are not ideal in practice. Even more, some components, such as DMSO in superabundance will inhibit the activities of the polymerase.

U.S. patent 5,646,019, the title of 'method for producing primed nucleic acid templates', introduced a method to accomplish the optimization of PCR amplification by adding heat-stable single-stranded nucleic acid binding protein (SSB) into the PCR system, wherein SSB protein only combines with single-stranded DNA instead of double-stranded DNA to inhibit the amplification of the non specific fragment comprising single standed DNA. However, the technique of purifying SSB is complicated, and the purity of the reagents is required high, so the cost of this method is very high. The commercial kit used in this method will be 6-7 times more expensive than common PCR reagents Furthermore, the PCR reagents should be reserved at -20°C to maintain the biological activities of single-stranded nucleic acid binding protein, and the active period of SSB is rather short

US-A-614061.3 relates to a PCR method for amplifying a gene using a metallic sample container. The sample container of US-A-6140613 is for heating a sample stored therein and includes a resin layer on the whole of the inner surface of the container, the container being made of metal oxide.

Patel, Hrishikesh E. et al., "Thermal conductivities of naked and monolayer protected metal nanoparticle based nanofluids: Manifestation of anomalous enhancement and chemical effects," Applied Physics Letters, Vol. 83, No. 14, pages 2931-2933, discloses the thermal conductivities of two kinds of Au nanoparticles, measured in water and toluene.

### DISCLOSURE OF THE INVENTION

An object of the present invention is to provide an optimized method of polymerase chain reaction amplification, by which the specificity of the reaction is enhanced, and the yield of the product is increased. The method is effective to optimize PCR system amplification in a simple and inexpensive manner, wherein the easily reserved material for optimizing can be conveniently removed from the PCR system Furthermore, the method of the present invention is applicable to a variety of PCR systems

Accordingly, to achieve above mentioned object, the present invention provides an optimized method of polymerase chain reaction amplification, comprising the step of adding any one of the following elemental materials for optimizing into PCR system to achieve the aim of optimizing PCR amplification: gold, titanium, nickel, bismuth, stibium, selenium, chromium, or the mixtures thereof In particular, the method comprises the following steps:
(1) Preparing elemental material for optimizing
   (i) The elemental material mentioned above is in the form of, for example, filament, sheet, particle, powder, colloidal or any other irregular forms According to the embodiments, the elemental materials in the form of filament, sheet, particle, powder or any other irregular forms may be obtained from the market, among which there are no particular requirements for the size of powder material for optimizing, and the size may be from the order of nanometer to micrometer As to material in colloidal form, it can be prepared by conventional method, whose size is not limited specifically.
      For example, to prepare colloidal gold, the following methods can be used: white phosphor reduction, sodium citrate reduction, sodium citrate-tannic acid reduction, etc.. Taking tri-sodium citrate reduction found in 1973 by Frens as an example to illustrate the preparation of colloidal gold, 001% HAuCl₄ is firstly heated till boiling, and then a mount of 1% tri-sodium citrate aqueous solution is immediately added. The color of the mixture solution is become Cambridge blue, and then changed into blue. The color is changed into red with continuous heating, and then into orange as boiling for 7 to 10 minutes At this time, the reaction is stopped All the above solution is prepared by the sterile pure water. The commercially available colloidal gold can be obtained from Sigma Corporation, wherein the size of colloidal gold is, for example, 5nm, 10nm, 20nm, with the concentration of 0.01% HAuCl₄, and no need to be diluted before it is used.
   (ii) The material in filament, sheet, or any other irregular forms is washed sequentially in sterile water and anhydrous ethanol for several times, and then air dried for later use. The material in particle or powder is washed sequentially in sterile water and anhydrous ethanol, and then dried in oven for later use. As to colloidal material, it does not need to be washed.
(2) The material in filament, sheet, particle, powder or any other irregular forms needs to be sterilized by drying method, wetting method, or ultraviolet radiation before it is used. There is no need to be sterilized for colloidal material before used
(3) Optimization of PCR system
   According to the present invention, the optimization of PCR system refers to adding a proper amount of any kind of elemental material for optimizing or the mixture mentioned above into PCR system to perform PCR amplification The proper amount refers to the amount of material for optimizing added into the PCR system The following is taken 25µL PCR system as an example to illustrate the amount of material for optimizing. It is easy to be understood that the amount of material for optimizing will be proportionally adjusted based on the amount used in 25µL system when the PCR system is greater or less than 25µL
   The material for optimizing in the form of filament, sheet or any other irregular forms is prepared with certain length and shape thus enabling them to be added into a PCR tube containing 25µL solution, wherein the material for optimizing is fully immersed in the solution and the liquid height of the solution is not higher than 0 6cm from the bottom of the PCR tube. If the liquid height is higher than 0.6cm, a uniform temperature distribution will be difficult to achieve. For elemental gold, the minimum amount based on the total surface area is at least 4mm²; for other elemental materials, the minimum amount based on the total surface area is at least 2mm² to get an effectively optimizing effect.
   The material in the form of particle should be added as one or more particles depending on its size into the PCR tube containing 25µL solution, wherein the material for optimizing is fully immersed in the solution and the liquid height of the solution is not higher than 0.6cm from the bottom of the PCR tube. It should be noted that the minimum amount of the material for optimizing added into the PCR system based on the total surface area is at least 2mm² to get an effectively optimizing effect.
   In the final 25µL PCR reaction system, the effective amount of 200-300 mesh titanium powder is ranged from 20µg to 1000µg, preferably from 50µg to 800µg. For chromium powder, the effective amount of 200 mesh chromium powder is ranged from 450µg to 1500µg, preferably from 600µg to 1200µg. Generally, the effective amount for other powder materials should be ranged from 50µg to 5000µg For gold powder, the effective amount is not less than 2mg. To obtain a precise amount, the elemental material in the form of powder should be weighted precisely, and then added into a proper amount of sterilized water, and fully suspended, thereafter, suspending solution in precise amount is immediately added into the PCR system, and finally adding water until 25µL
   In the final 25µL PCR system, the effective amount of colloidal gold is ranged from 0.06µL to 6.0µL. Particularly, the effective amount of 5nm size of colloidal gold with 0.01% HAuCl₄ concentration is 0.06µL-0.5µL, and preferably is 0.08µL-0.16µL. The effective amount of 10nm size of colloidal gold with 0.01% HAuCl₄ concentration is 0.5µL-2.5µL, and preferably is 0.5µL-16µL. The effective amount of 20nm size of colloidal gold with 0.01% HAuCl₄ concentration is 0.8µL-60µL, and preferably is 0 .8µL-3.5µL. For other materials for optimizing in colloidal form, they are prepared by conventional methods or obtained by purchase, and in the case of conventional concentration, the effective amount is 0.5µL-4µL
   In the final 25µL PCR system, any of the elemental materials mentioned above can be used in combination. The amount of each material in a mixture is varied with the sort of the material and its form For the mixture in powder form, the suitable amount is ranged from 50µg to 5000µg; for the mixture in colloidal form, the suitable amount is 0.06µL-6.0µL; and for the mixture of powder and colloidal materials, the total suitable amount of powder materials is from 20µg to 5000µg and the total suitable amount of colloidal materials is 0.02µL-6.0µL.
   The PCR amplification is performed according to conventional procedures, for example as follows: 94°C or 2 minutes (pre-heating), followed by 30-45 cycles: 94 °C for 30 seconds (denaturing), 58°C for 1~3 minutes (annealing), and 72°C for 45 seconds~3 minutes (extension), followed by a final extension at 72 °C for 7~10 minutes. The number of cycles, the time of annealing, and the time of extension are varied with the purpose of amplification, the object to be amplified and the primers.
(4) Analyzing the PCR products
   The agarose gel electrophoresis procedure is used to check the PCR products, and the optimizing effects can be shown by comparing the optimized sample with the control sample.
(5) Removing the materials for optimizing from the PCR system
   To collect the PCR products, the materials for optimizing are needed to be removed from the PCR system. For those materials in the form of filament, sheet, and any other irregular forms, the solution can be directly absorbed out to separate from the materials for optimizing; for those in the form of powder and particle, the PCR system is centrifuged at a low speed and the supernatant can be harvested; for colloidal materials, the PCR system is firstly frozen overnight at a temperature of -20°C, and then is centrifuged at a high speed after thawing to obtain the supernatant.

PCR in this invention refers to various kinds of PCR including common PCR, selective amplification of specific low copy DNA in highly complex genome template, long distance PCR, multiplex PCR, single molecule PCR, two-round PCR after the imperfect first round PCR, rapid PCR and so on.

Materials for optimizing mentioned in this invention such as gold are also applicable to optimize other reactions such as polymerase extension reaction with the similar mechanism to PCR reaction, and other biochemical methods based on PCR method.

For PCR systems, they are recorded in the manufacturer's instructions of the various commercial polymerases. Taking Ex Taq polymerase (Takara Corporation) as an example, the composition of the PCR system is as follows:

| | |
|---|---|
| Takara Ex Taq polymerase (5U/µL) | 0.25 µL |
| 10× PCR buffer solution | 2.5 µL |
| dNTP substrate (10mM) | 0.75 µL |
| Mg²⁺ (250mM) | 0.35 µL |
| Primer 1 (1µM) | 2.5 µL |
| Primer 2 (1µM) | 2.5 µL |
| Template | 1 µL |
| H₂O | added up to 25µL |

"analyzing the PCR products" or the similar description in this invention refers to the detection of the PCR products by conventional techniques, for example, the PCR products are analyzed by agarose gel electrophoresis or capillary electrophoresis

The agarose gel electrophoresis is performed according to the conventional techniques comprising the following steps:
1) preparing 15% agarose gel (containing ethidium bromide);
2) loading different PCR products into electrophoresis cells and molecular marker as reference;
3) electrophoresis at 4~5V/cm voltage;
4) analysis the results by ultraviolet detection with gel image system.

In short, the amplification method of the present invention is more effective than the conventional amplification method. Furthermore, the amplification method of the present invention could be widely employed in many fronts, and the materials for optimizing are simple to be removed from the PCR system. And more importantly, the materials for optimizing used in the present invention, such as elemental gold, elemental titanium, elemental bismuth, elemental nickel, elemental stibium, elemental selenium, elemental chromium and their mixture would not cause any inhibition of the polymerase, and could be reserved for long term at a temperature of 4°C without losing their activity.

From another perspective, the method for amplification of the present invention is cheaper than the conventional methods. For example, the commercially available titanium powder costs only one hundredth price of the single stranded binding protein, so no need to be prepared. The commercially available colloidal gold is also cheaper than single stranded binding protein, and meanwhile easy to be preserved. Additionally the colloidal gold has the advantages of good uniformity and precise loading. The optimized PCR system of this invention can not only be applied to PCR instruments with temperature precisely controlled but also to PCR instruments with temperature imprecisely controlled The optimized method of this invention is applicable to various PCR amplifications, polymerase extension and other biochemical methods based on PCR method. In particular, it is applicable to PCR system which is difficult to be amplified such as low copy PCR, long distance PCR, multiplex PCR, single molecule PCR, two-round PCR after the imperfect first round PCR. It is also applicable to rapid PCR. The optimized method of this invention has great potential value in the fields of gene detection and gene clone, genetic analysis, clinical diagnosis, gene chip and new materials etc..

### BRIEF DESCRIPTION OF THE DRAWINGS

FIG. 1 illustrates the flow chart of the optimized method of the present invention.
FIG. 2 illustrates the optimized effect of gold sheets on common PCR amplification.
FIG. 3 illustrates the optimized effect of gold plated film on serial diluted low copy template PCR amplification.
FIG. 4 illustrates the optimized effect of colloidal gold on single molecule PCR amplification.
FIG. 5 illustrates the optimized effect of colloidal gold on multiplex PCR amplification.
FIG. 6 illustrates the optimized effect of colloidal gold on long distance PCR amplification
FIG. 7 illustrates the optimized effect of colloidal gold in different sizes on two-round PCR amplification.
FIG. 8 illustrates the optimized effect of colloidal gold on two-round PCR amplification.
FIG. 9 illustrates the optimized effect of titanium powder on common PCR amplification.
FIG. 10 illustrates the optimized effect of titanium powder on two-round PCR amplification.
FIG. 11 illustrates the optimized effect of titanium filament on multiplex PCR amplification
FIG 12 illustrates the optimized effect of elemental nickel, elemental bismuth, elemental stibium, elemental selenium and elemental chromium on two-round PCR amplification.
FIG. 13 illustrates the optimized effect of chromium powder on two-round PCR amplification.
FIG. 14 illustrates the effect of rapid PCR by using colloidal gold as material for optimizing.
FIG. 15 illustrates the optimized effect of the mixture on two-round PCR amplification.

### DETAILED DESCRIPTION OF PREFERRED EMBODIMENTS OF THE INVENTION

The examples provided hereinafter assist in better understanding the present invention, which are not intended to limit the present invention.

### Example 1 Optimizing common PCR by using gold foil

The optimized method in this example is used to optimize the PCR amplification with non-specific amplification products. The method comprises the following steps:
1. Preparing PCR system
the composition is as follows:

| | |
|---|---|
| Takara Ex Taq polymerase (5U/µL) | 0.25 µL |
| 10× PCR buffer solution | 2.5 µL |
| dNTP substrate (10mM) | 0.75 µL |
| Mg²+(250mM) | 0.35 µL |
| Primer 1 (1µM) | 2.5 µL |
| Primer 2 (1µM) | 2.5 µL |
| Template | 1 µL |
| H₂O | added water up to 25µL after the addition of the material for optimizing |

In this example, λDNA (purchased from Hua Mei bioengineering Company) is used as template in PCR. Ex Taq polymerase is purchased from Takara Corporation. The fragment to be amplified has the length of 283bp. The two primers are as follows: P1: 5'-GGCTTCGGTCCCTTCTGT-3', P2: 5'-CACCACCTGTTCAAACTCTGC-3'.
2. To the above PCR system, washed and sterilized gold foil with the size of 2mm×2mm as material for optimizing was added. For each 25 µL system, at least one piece of gold foil was added. Meanwhile, the system without gold foil was as a control.
3. Amplification of the template molecule
The amplification condition is as follows: 94°C for 2 minutes (pre-heating), followed by 30 cycles of 94°C for 30 seconds (denaturing), 58°C for 1 minute (annealing) and 72°C for 45 seconds (extension); followed by a final extension at 72°C for 7 minutes.
4. The PCR products are analyzed by agarose gel electrophoresis.

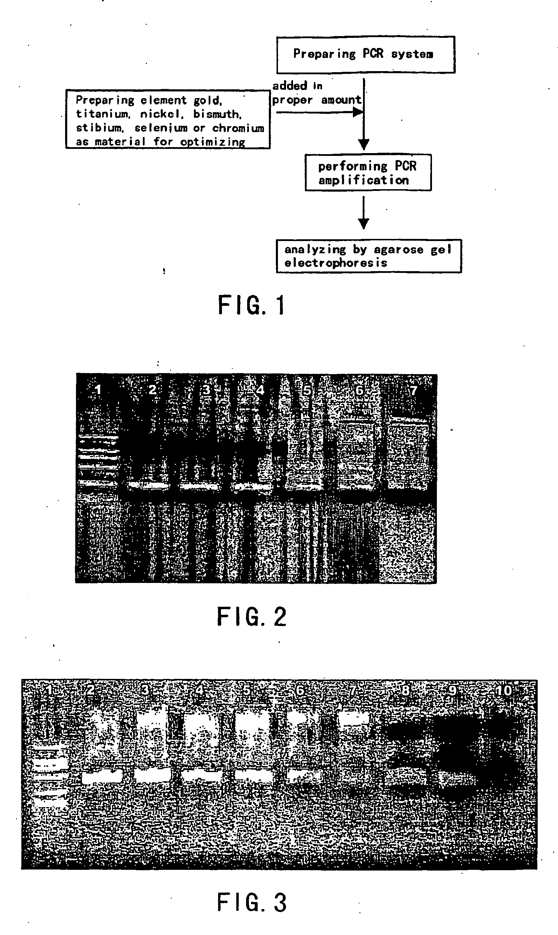

The amplification result is shown in FIG. 2. From left to right: lane 1 is DNA Molecular Weight Marker (DL2000 from Takara corporation, 2000bp, 1000bp, 750bp, 500bp, 250bp, and 100bp); lanes 2, 3, and 4 are three parallel samples of the system containing gold foil; lanes 5, 6 and 7 are three parallel samples of the conventional system. It can be seen from the figure that the PCR amplification results show very few non-specific amplification in the systems containing the gold foil (lane 2, 3, and 4). However, the PCR amplification results show significant non-specific PCR amplification in the systems without gold foil with severe smear (lanes 5, 6 and 7). Notably, an increase in specificity by using gold foil is seen in this experiment.

### Example 2 Optimizing serial diluted low copy template PCR by using gold plated film

The optimized method for serial diluted low copy template PCR comprises the following steps:
1. Serial diluting the DNA molecule of plasmid pBR322 to obtain the templates in series copy numbers. The copy numbers are from 10⁵ copies to theoretically close to single-copy.
2. Preparing PCR system whose composition is similar with that of example 1 except the following differences: the template is changed into pBR322 with various dilution times, and the fragment to be amplified has the length of 342bp. Addtionally, the primers are: P1: 5'-CTAACGGATTCACCACTCCAAGAA-3'; P2: 5'-GACTTCCGCGTTTCCAGACTTTAC-3'.
3. For the PCR systems with the copy numbers of 10⁵, 10⁴, 10³ and 10² copies, no material for optimizing is added. Each one is as a control. For the system with the copy number theoretically close to single-copy, three samples are prepared for the following experiments: One sample is taken as a control wherein no material for optimizing is added; and the other two samples are added into washed and sterilized gold plated film as the material for optimizing. To each 25µL system, one piece of gold plated film is added. For the gold plated film, the base is made of silicon, and one side is plated by gold with the area of 1.5mmX 4mm.
4. To amplify the template molecules under the condition as follows: 94°C for 2 minutes (pre-heating), followed by 45 cycles of 94°C for 30 seconds (denaturing), 58°C for 1 minute (annealing) and 72°C for 45 seconds (extension); followed by a final extension at 72°C for 7 minutes.
5. The PCR products are analyzed by agarose gel electrophoresis.

The amplification results are shown in FIG. 3. From left to right: lane 1 is DNA Molecular Weight Marker (DL2000 from Takara corporation, 2000bp, 1000bp, 750bp, 500bp, 250bp, and 100bp); lane 2 is the result of the PCR containing 10⁵ copies of pBR322 plasmids as template; lane 3 is the result of the PCR containing 10⁴ copies of pBR322 plasmids as template; lane 4 is the result of the PCR containing 10³ copies of pBR322 plasmids as template; lane 5 is the result of the PCR containing 10² copies of pBR322 plasmids as template; lane 6 is the result of the PCR containing 10 copies of pBR322 plasmids as template; lanes 7, 8 and 9 are the results of the PCR containing nearly one copy number of pBR322 plasmid as template; lane 10 is the result of blank negative control. For the PCR systems of lanes 2 to 7, no materials for optimizing are added. While for the PCR systems of lanes 8 to 10, the gold plated film is added. The experiment is designed such that no material for optimizing is added into the system containing template with high copy number while the material for optimizing is added into the system containing very low copy number of template. It can be seen from FIG. 3, the results of PCR without gold plated films (lanes 2-7) show severe smear, while the results of single copy PCR containing gold plated films (lanes 8 and 9) show single clear band due to the elimination of non-specific amplification. Notably, an increase in specificity by using gold plated film is seen from this example.

### Example 3 Optimizing single molecule PCR by using colloidal gold

The optimized method for single molecule PCR amplification comprises the following steps:
1. The template used in this example is a single DNA molecule prepared from pBR322 plasmid (purchased from Hua Mei bioengineering Company) by nanomanipulation with the help of atomic force microscopy (AFM). The single molecule precisely selected is used to prepare the single copy template solution.
2. Preparing PCR system whose composition is similar with that of example I except the following difference: The template is changed into single copy template solution. The length of the fragment to be amplified and the primers are the same with those of example 2.
3. To the above PCR system, colloidal gold (purchased from Sigma Corporation, 10nm, 0.01% HAuCl₄) is added. For each 25µL system, the amount of colloidal gold is 1 µL. Meanwhile, the system without any material for optimizing is as a control.
4. The amplification is performed under the condition as follows: preheating at 94°C for 2 minutes; 10 cycles of 94°C for 30 seconds (denaturing), 65°C for 3 minutes (annealing) and 72°C for 45 seconds (extension); 10 cycles of 94°C for 30 seconds (denaturing), touchdown from 65°C to 58°C for 1 minute (annealing) and 72°C for 45 seconds (extension); 30 cycles of 94°C for 30 seconds (denaturing), 58°C for 1 minute (annealing) and 72°C for 45 seconds (extension); finally, additional extension at 72°C for 7 minutes.
5. The PCR products are analyzed by agarose gel electrophoresis.

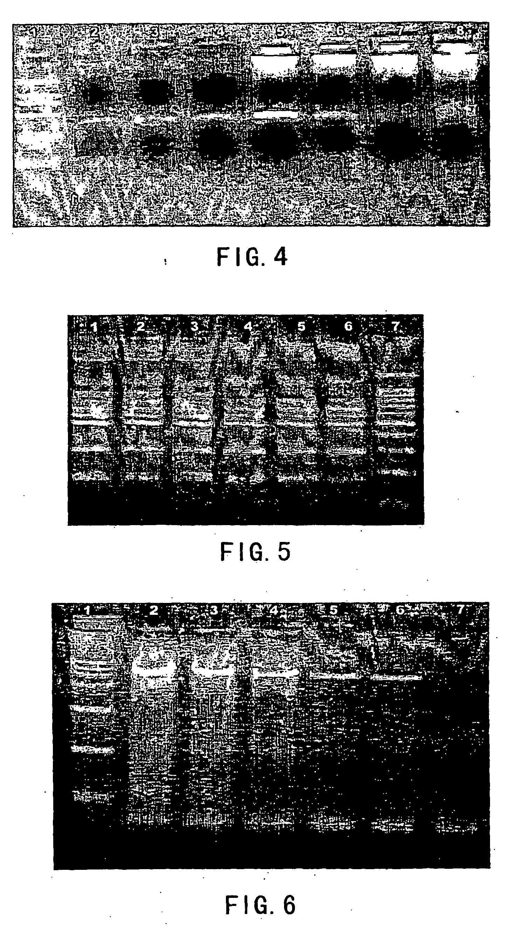

The amplification results are shown in FIG. 4. From left to right: lane 1 is DNA Molecular Weight Marker (DL2000 from Takara corporation, 2000bp, 1000bp, 750bp, 500bp, 250bp, and 100bp); each reaction of lanes 2, 3, and 4 contains single molecule DNA template and I µL colloidal gold; reaction of lane 5 contains 10 molecules as template but no colloidal gold; reaction of lane 6 contains single molecule DNA but no colloidal gold; reactions of lane 7 and 8 contain neither template DNA nor colloidal gold as a blank negative control. The amplification result of PCR containing colloidal gold shows a single predominant band (lanes 2, 3, and 4). However, the amplification result of PCR without colloidal gold (lanes 5 and 6) shows non-specific PCR products with severe smear. Notably, an increase in specificity by using colloidal gold is seen in this experiment.

### Example 4 Optimizing multiplex PCR by using colloidal gold

The optimized method for multiplex PCR amplification comprises the following steps:
1. Preparing PCR system whose composition is similar with that of example 1 except that eight pairs of primers are used in this example to perform multiplex PCR amplification. To amplify the DNA molecules respectively with the lengths of 159bp, 283bp, 485bp, 573bp, 660bp, 785bp, 942bp and 1240bp, the sequences of the eight primers used are:
   Pair 1: P1: 5'-GGATGACCCCTCCAGCG-3'; P2: 5'-CCGTAAACTCCACCCTTCG-3'
   Pair 2: P1: 5'-GATGCTTGAACCCGCCTAT-3'; P2: 5'-GCCTGTCGTGGTCCGTC-3'
   Pair 3: P1: 5'-GGCTTCGGTCCCTTCTGT-3'; P2: 5'-CACCACCTGTTCAAACTCTGC-3'
   Pair 4: P1: 5'-TGGAGCGTGAGGAATGGG-3'; P2: 5'-GCCGTGTTCGGGTAGCA-3'
   Pair 5: P1: 5'-CTCGCTCATAACAGACATTCACT-3';
      P2: 5'-TCAACATCTTCTCGGGCATA-3'
   Pair 6: P1:5'-GCACAAGTCCGACAACCC-3';
      P2: 5'-GCTGAGGAGATAAATAATAAACGAG-3'
   Pair 7: P1:5'-CAAAACTAAGGGCATAGACAATAA-3';
      P2: 5'-TGGTTCAGAAGATAAATCGCTC-3'
   Pair 8: P1: 5'-CGGAACATCTCGGTAACTGC-3'; P2: 5'-CGTCGCTGTCTCGCCAC-3'
2. To the above PCR system, colloidal gold (purchased from Sigma Corporation, 10nm, 0.01% HAuCl₄) is added. For each 25µL system, the amount of colloidal gold is 1 µL. Meanwhile, the system without any material for optimizing is as a control.
3. The amplifications are performed under the condition as Example 1.
4. The PCR products are analyzed by agarose gel electrophoresis.

The results are shown in FIG. 5. From left to right: lanes 1, 2, and 3 show the results of three parallel samples of conventional PCR system; lanes 4, 5, and 6 show the results of three parallel samples of PCR system containing colloidal gold; and lane 7 is DNA Molecular Weight Marker (1500bp, 1000bp, 900bp, 800bp, 700bp, 600bp, 500bp, 400bp, 300bp, 200bp, and 100bp, from top to below). The amplification results of PCR containing colloidal gold show clear bands (lanes 4, 5, and 6). However, the amplification results of PCR without colloidal gold (lanes 1, 2, and 3) show non-specific PCR products with smear. Notably, an increase in specificity by using colloidal gold is seen in this experiment.

### Example 5 Optimizing long distance PCR amplification by using colloidal gold

The optimized method for long distance PCR amplification comprises the following steps:
1. Preparing PCR system whose composition is similar with that of example 1. In this example, the following two primers are used to amplify a 4200bp fragment.
   P1: 5'-ACGCTCGTCGTTTGGTATGGC-3'; P2: 5'-CCGGCTGGCTGGTTTATTGC-3'
2. To the above PCR system, colloidal gold (purchased from Sigma Corporation, 10nm, 0.01% HAuCl₄) is added. For each 25µL system, the amount of colloidal gold is 1 µL. Meanwhile, the system without any material for optimizing is as a control.
3. The amplifications are performed as follows: 40 cycles of 95°C for 2 minutes (pre-heating), 94°C for 30 seconds (denaturing), 58°C for 1 minute (annealing) and extend at 72°C for 3 minutes; followed by a final extension at 72°C for 10 minutes.
4. The PCR products are analyzed by agarose gel electrophoresis.

The results are shown in FIG. 6. From left to right: lane 1 is DNA Molecular Weight Marker DL 15,000 (Takara corporation, consisted of 15,000bp, 10,000bp, 7,500bp, 5,000bp, 2,500bp, 1,000bp, and 250bp from top to below); lanes 2, 3 and 4 show the results of conventional reaction systems not containing colloidal gold; lanes 5 and 6 show the results of the systems containing colloidal gold as optimized material; and lane 7 is the result of the blank negative control. The amplification result of PCR containing colloidal gold shows a single predominant band corresponding to the 4200bp target (lanes 5 and 6). However, the amplification results of PCR without colloidal gold (lanes 1, 2, and 3) show non-specific PCR products with broad dispersion. Notably, an increase in specificity by using colloidal gold is seen in this experiment.

### Example 6 Optimizing two-round PCR by using colloidal gold in different sizes

The optimized method for two-round PCR amplification comprises the following steps:
1. Preparing PCR system whose composition is similar with that of example 1 except that the template in this example is the first round PCR product. To amplify the DNA fragment of 283bp, the same primer sequences as example 1 are used.
2. To the above PCR system, colloidal gold in 5nm, 10nm, and 20nm (purchased from Sigma Corporation, 0.01% HAuCl₄) is respectively added. For each 25µL system, the increased amount in gradient of colloidal gold are added according to different sizes. Meanwhile, the corresponding systems without any materials for optimizing are as controls.
3. The amplifications are performed under the same condition as example 1.
4. The PCR products are analyzed by agarose gel electrophoresis.

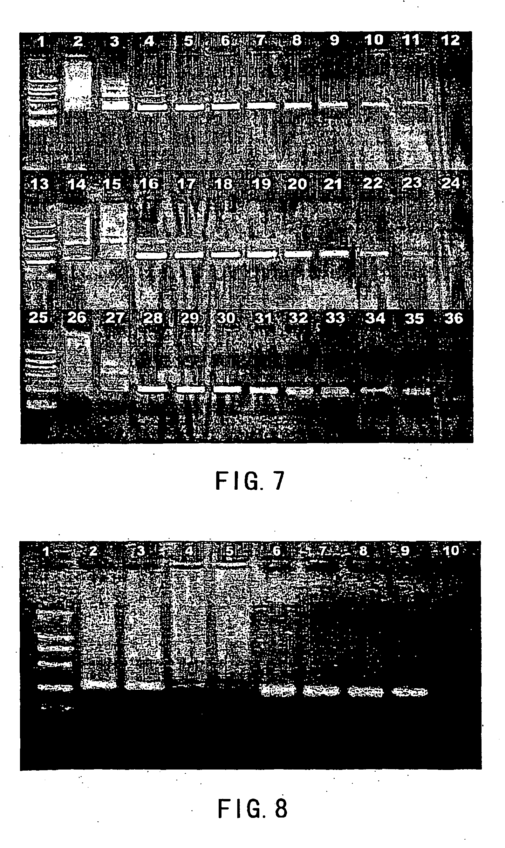

The results are shown in FIG. 7. Lanes 1,13 and 25 are DNA Molecular Weight Marker (DL2000, Takara Corporation, 2000bp, 1000bp, 750bp, 500bp, 250bp, and 100bp); the reactions of lanes 2, 14 and 26 do not contain colloidal gold; lanes 12, 24, and 36 are the results of the negative control. The amounts of 5nm colloidal gold contained in reactions are as below: 0.03µL (lane 3), 0.06µL (lane 4), 0.08µL (lane 5), 0.1µL (lane 6), 0.12µL (lane 7), 0.14µL (lane 8), 0.16µL (lane 9), 0.25µL (lane 10), and 0.5µL (lane 11). The amounts of 10nm colloidal gold contained in reactions are as below: 0.2µL (lane 15), 0.5µL (lane 16), 0.6µL (lane 17), 0.8µL (lane 18), 1.0µL (lane 19), 1.3µL (lane 20), 1.6µL (lane 21), 2.0µL (lane 22), and 2.5µL (lane 23). The amounts of 20nm colloidal gold contained in reactions are as below: 0.5µL (lane 27), 0.8µL (lane 28), 1.5µL (lane 29), 2.0µL (lane 30), 2.5µL (lane 31), 3.0µL (lane 32), 3.5µL (lane 33), 4.5µL (lane 34), and 6.0µL (lane 35). Each reaction system is 25µL. The amplification results of PCR containing colloidal gold show a single predominant band. However, the amplification results of PCR without colloidal gold show non-specific PCR products with smear. Notably, an increase in specificity by using colloidal gold is seen in this experiment.

The amounts of different size colloidal gold needed to optimize the reaction are different. The effective amount of 5nm colloidal gold is from 0.06µL to 0.5µL, and the preferred amount of 5nm colloidal gold is from 0.08µL to 0.16µL. The effective amount of 10nm colloidal gold is from 0.5µL to 2.5µL, and the preferred amount of 10nm colloidal gold is from 0.5µL to 1.6µL. The effective amount of 20nm colloidal gold is from 0.8µL to 6.0µL, and the preferred amount of 20nm colloidal gold is from 0.8µL to 3.5µL.

### Example 7

Optimizing two-round PCR by using colloidal gold and quantifying the target product of PCR.

The optimized method for two-round PCR amplification comprises the following steps:
1. Preparing PCR system whose composition is similar with that of example 1 except that the template in this example is the first round PCR product. To amplify the DNA fragment of 283bp, the same primer sequences as example I are used.
2. To the above PCR system, colloidal gold in 10nm (purchased from Sigma Corporation, 0.01% HAuCl₄) is added. For each 25µL system, the amount of colloidal gold solution added is 1µL. Meanwhile, the corresponding system without any materials for optimizing is as a control.
3. The amplifications are performed under the same condition as example 1.
4. The PCR products are analyzed by agarose gel electrophoresis.

The results are shown in FIG. 8. From left to right: lane 1 is DNA molecular weight marker (DL2000, Takara Corporation, 2000bp, 1000bp, 750bp, 500bp, 250bp, and 100bp); lanes 2, 3, 4 and 5 show the results of four parallel samples without colloidal gold; lanes 6, 7, 8 and 9 show the results of four parallel samples containing colloidal gold as material for optimizing; lane 10 is the result of the negative control. The amplification results of PCR containing colloidal gold show a single predominant band (lanes 6, 7, 8 and 9). However, the amplification results of PCR without colloidal gold show non-specific PCR products having severe smear (lanes 2, 3, 4 and 5). The electrophoresis lanes are analyzed by photodensitometry, and the results show that the product yields of lanes 6, 7, 8 and 9 are higher respectively by 0.8, 0.5, 0.2 and 0.2 times than the yield of lane 2. Notably, an increase in specificity and yield by using colloidal gold is seen in this experiment.

### Example 8 Optimizing common PCR by using titanium powder

The optimized method for common PCR to enhance the specific amplification comprises the following steps:
1 Preparing PCR system whose composition is the same as that of example 1. The template is λDNA which purchased from Hua Mei Bioengineering Company. Ex Taq polymerase is purchased from Takara Corporation. The DNA fragment to be amplified has the length of 283bp. In this example, two primers are used as follows:
   P1: 5'-GGCTTCGGTCCCTTCTGT-3', P2: 5'-CACCACCTGTTCAAACTCTGC-3'
2 To the above PCR system, colloidal gold (purchased from Sigma Corporation, 10nm 001% HAuCl₄) or washed and sterilized elemental titanium powder (purchased from Shanghai Chemical Reagent Co. Ltd., 200-300 mesh) is added. For each 25µL system, the amount of colloidal gold is 1 µL, and the titanium powder is respectively 100µg, 400µg and 800µg. Meanwhile, the system without any material for optimizing is as a control.
3 The amplifications are performed under the condition as example 1.
4 The products of PCR reactions are analyzed by agarose gel electrophoresis .

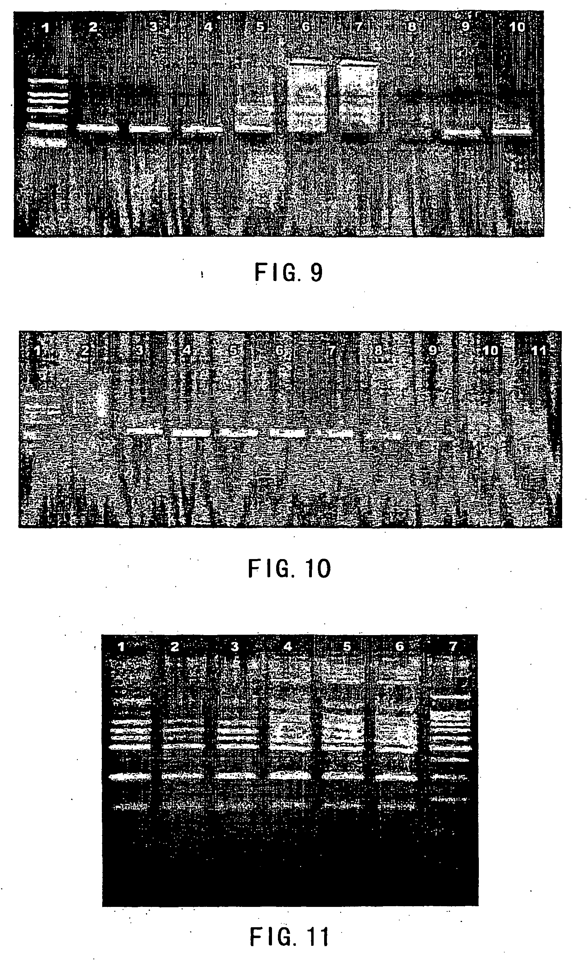

The amplification results are shown in FIG. 9 From left to right: lane I is DNA molecular weight marker (DL2000, Takara Corporation, 2000bp, 1000bp, 750bp, 500bp, 250bp, and 100bp); each reaction of lanes 2, 3 and 4, the three parallel samples, contains 1µL colloidal gold; each reaction of lanes 5, 6 and 7, the three parallel samples, does not contain any materials for optimizing; reactions of lanes 8, 9 and 10 contain 800µg, 400µg and 100µg titanium powder, respectively. The amplification results of PCR containing colloidal gold (lanes 2, 3 and 4) and titanium powder (lanes 9 and 10) show a single predominant band. However, the amplification results of PCR without any materials for optimizing show non-specific PCR products with smear. By comparison of the results of lanes 8, 9 and 10, 100µg and 400µg titanium powder optimizes PCR well while 800µg titanium powder exhibits the inhibition effect on PCR to some extent although showing a single band.

### Example 9 Optimizing two-round PCR by using titanium powder

The optimized method for two-round PCR in this example comprises the following steps:
1. Preparing PCR system whose composition is similar with that of example 1 except that the template in this example is the first round PCR product. The DNA fragment to be amplified has the length of 283bp, and the same primer sequences as example 1 are used.
2. To the above PCR system, various amounts of washed and sterilized titanium powder (purchased from Shanghai Chemical Reagent Co. Ltd., 200-300 mesh) are added. For each 25µL system, the amounts of titanium powder are added with 20µg, 50µg, 100µg, 200µg, 300µg, 400µg, 600µg and 800µg, respectively. Meanwhile, the corresponding systems without any materials for optimizing are as controls.
3. The amplifications are performed under the same condition as example 1.
4. The PCR products are analyzed by agarose gel electrophoresis.

The results are shown in FIG. 10. From left to right: lane 1 is DNA molecular weight marker (DL2000, Takara Corporation, 2000bp, 1000bp, 750bp, 500bp, 250bp, and 100bp); the reaction of lane 2 is conventional PCR system which does not contain any materials for optimizing, and is used as positive control; lanes 3-10 are the results of the systems containing various amounts of titanium powder, and the amounts of titanium powder are respectively as below: 20µg (lane 3), 50µg (lane 4), 100µg (lane 5), 200µg (lane 6), 300µg (lane 7), 400µg (lane 8), 800µg (lane 9) and 1000µg (lane 10); the reaction of lane 11 is negative control. The amplification results of PCR containing suitable amount of titanium powder show a single predominant band. However, the amplification results of PCR without titanium powder show non-specific PCR products having severe smear. The effective amount of titanium powder is from 20µg to 1000µg, and the preferred amount of titanium powder is from 50µg to 800µg.

### Example 10 Optimizing multiplex PCR by using titanium filaments

The optimized method for multiplex PCR amplification comprises the following steps:
1. Preparing PCR system whose composition is similar with that of example 1 except that eight pairs of primers are used in this example to perform multiplex PCR amplification. To amplify the DNA molecules respectively with the lengths of 159bp, 283bp, 485bp, 573bp, 660bp, 785bp, 942bp and 1240bp, the sequences of the primers used are the same as those of example 4.
2. To the above PCR systems, washed and sterilized titanium filaments with 3mm in length, and 0.5mm in diameter are added. Meanwhile, the corresponding systems without any materials for optimizing are as controls.
3. The amplifications are performed under the condition as Example 1
4. The PCR products are analyzed by agarose gel electrophoresis.

The results are shown in FIG. 11 From left to right: reactions of lanes 1, 2, and 3 contain titanium filaments; reactions of lanes 4, 5, and 6 do not contain any materials for optimizing; and lane 7 is DNA molecular weight marker (1500bp, 1000bp, 900bp, 800bp, 700bp, 600bp, 500bp, 400bp, 300bp, 200bp, and 100bp, from top to below). The amplification results of PCR containing titanium filaments show clear bands. However, the amplification results of PCR without the material for optimizing show non-specific PCR products with smeat. Notably, an increase in specificity by using titanium filament is seen in this experiment.

### Example 11

Optimizing two-round PCR by using elemental nickel, elemental bismuth, elemental stibium, elemental selenium and elemental chromium as the materials for optimizing

The optimized method for two-round PCR in this example comprising the following steps:
1. Preparing PCR system whose composition is similar with that of example 1 except that the template in this example is the first round PCR product. The DNA fragment to be amplified has the length of 283bp, and the primer sequences used are the same as those of example 1.
2. To the above PCR systems, washed and sterilized elemental nickel, elemental bismuth, elemental stibium, elemental selenium and elemental chromium are added. Meanwhile, the corresponding systems without any materials for optimizing are as controls.
3. The amplifications are performed under the same condition as example 1.
4. The PCR products are analyzed by agarose gel electrophoresis.

The nickel filaments used in this example is 3.2mm in length, and 0.8mm in diameter; the bismuth particles used in this example are spherical particles with 0.1-0.2mm in diameter, and 12mg in weight for each system; the stibium particles used in this example are spherical particles with 0.1-0.2mm in diameter, and 30mg in weight for each system; the amount of selenium powder used in this example for each system is 400µg; and the amount of chrome powder used in this example for each system is 800µg.

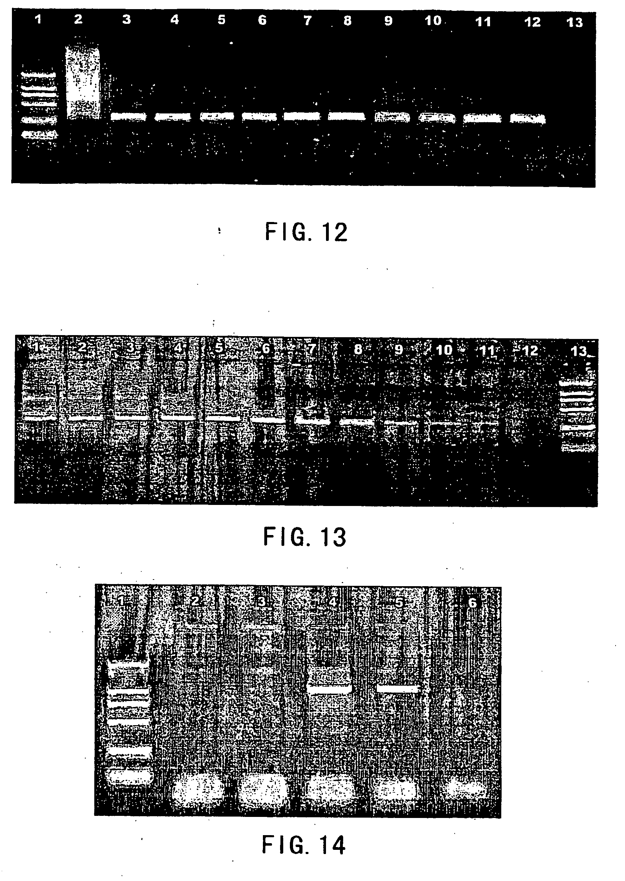

The results are shown in FIG. 12. Lane is DNA molecular weight marker; the reaction of lane 2 is the conventional PCR system without any materials for optimizing, and is as a positive control; the materials for optimizing contained in reactions are as below: nickel filaments (lanes 3 and 4), bismuth particles (lanes 5 and 6), stibium particles (lanes 7 and 8), selenium powder (lanes 9 and 10), and chrome powder (lanes 11 and 12); and lane 13 is the negative control. The amplification results of PCR containing elemental titanium, elemental nickel, elemental bismuth, elemental stibium, elemental selenium and elemental chromium show clear bands. However, the amplification results of conventional PCR system without any materials for optimizing show non-specific PCR products with obvious smear. Notably, an increase in specificity by using elemental nickel, elemental bismuth, elemental stibium, elemental selenium and elemental chromium is seen in this experiment.

### Example 12 Optimizing two-round PCR by using chromium powder

The optimized method for two-round PCR in this example comprising the following steps:
1. Preparing PCR system whose composition is similar with that of example 1 except that the template in this example is the first round PCR product. The DNA fragment to be amplified has the length of 283bp, and the primer sequences used are the same as those of example 1
2. To the above PCR systems, washed and sterilized elemental chromium powder (200 mesh, Shanghai Chemical Reagent Co Ltd.) is added. For each 25µL reaction system, the amount of elemental chromium powder are respectively 150µg, 300µg, 450µg, 600µg, 750µg, 900µg, 1000µg, 1200µg, 1500µg and 2000µg. Meanwhile, the corresponding systems without any materials for optimizing are as controls.
3. The amplifications are performed under the same condition as example 1.
4. The PCR products are analyzed by agarose gel electrophoresis.

The results are shown in FIG. 13. From left to right: lane 1 shows the result of conventional system without any materials for optimizing, which is as positive control; the amount of chromium powder contained in each 25µL reaction system is as below: 150µg (lane 2), 300µg (lane 3), 450µg (lane 4), 600µg (lane 5), 750µg (lane 6), 900µg (lane 7), 1000µg (lane 8), 1200µg (lane 9), 1500µg (lane 10) and 2000µg (lane 11); lane 12 is the negative control, and lane 13 is DNA molecular weight marker (DL2000, Takara Corporation, 2000bp, 1000bp, 750bp, 500bp, 250bp, and 100bp). The amplification results of PCR containing chromium powder show a single predominant band with the decrease of non-specific amplification products. However, the amplification results of PCR as positive controls show non-specific PCR products with severe smear. The effective amount of chromium powder is 450µg to 1500µg, and the preferred amount of chromium powder is 600µg to 1200µg. The result shows the notably optimized effect of optimization in this experiment.

### Example 13 Achieving rapid PCR amplification by using colloidal gold

The achieve rapid PCR amplification, the optimized method comprises the following steps:
1. Preparing PCR system whose composition is shown in example 1, wherein DNA of *Escherichia coli* (purchased from BioDev-Tech. Scientific & Technical Co. Ltd.) is used as template. For Ex Taq polymerase, it is purchased from Takara Corporation. The fragment to be amplified has the length of 997-bp. The sequences of the primers used are as below:
   P1: 5'-CCAGCAGCCGCGGTAATACG-3'; P2: 5'-ATCGGTTACCTTGTTACGACTTC-3'.
2. To the above PCR systems, the treated colloidal gold in 10nm (purchased from Sigma Corporation, 0.01% HAuCl₄) is added. For each 25µL system of the two samples, 0.5µL colloidal gold solution is added. Meanwhile, the two corresponding systems without any materials for optimizing are as controls.
3. The rapid PCR is performed as follows: 30 cycles of 98°C for 1 second, 60°C for 15 seconds (annealing); after the 30 cycles, an extension at 72°C for 5 minutes is performed.
4. The products of PCR reactions are analyzed by agarose gel electrophoresis.

The results are shown in FIG. 14. From left to right: lane 1 is DNA molecular weight marker (DL2000, Takara Corporation, 2000bp, 1000bp, 750bp, 500bp, 250bp, and 100bp); lanes 2 and 3 show the results of two parallel samples of conventional systems without any materials for optimizing; lanes 4 and 5 show the results of two parallel samples containing 0.5µL colloidal gold; reaction of lane 6 is the negative control in which no template is contained. The amplification results of rapid PCR containing colloidal gold (lanes 4 and 5) show a single predominant band corresponding to the target. However, the amplification results of PCR without colloidal gold (lanes 2 and 3) do not show the target band. In this example of rapid PCR, colloidal gold enhances the specificity and efficiency, shortens the reaction time and increases the yields of target products. Rapid PCR is achieved with the help of colloidal gold.

### Example 14 Optimizing two-round PCR by using the mixtures as materials for optimizing The optimized method for two-round PCR comprises the following steps:

1. Preparing PCR system whose composition is similar with that of example 1 except that the template in this example is the first round PCR product. The DNA fragment to be amplified has the length of 283bp, and the primer sequences used are the same as those of example 1.
2. To the above PCR system, various amounts of mixtures as the materials for optimizing are added. The said mixtures are the mixture of chromium powder (200 mesh, purchased from Shanghai Chemical Reagent Co. Ltd.) and titanium powder (200-300 mesh, purchased from Shanghai Chemical Reagent Co. Ltd.); and the mixture of colloidal gold (10 nm, 0.01%HAuCl₄, purchased from Sigma Corporation) and titanium powder (200-300 mesh, purchased from Shanghai Chemical Reagent Co. Ltd.). Meanwhile, the corresponding systems without any materials for optimizing are as controls.
3. The amplifications are performed under the same condition as example 1.
4. The PCR products are analyzed by agarose gel electrophoresis.

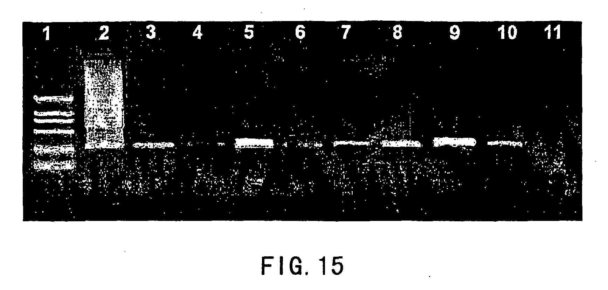

The results are shown in FIG. 15. From left to right: lane 1 is DNA molecular weight marker (DL2000, Takara Corporation, 2000bp, 1000bp, 750bp, 500bp, 250bp, and 100bp); the reaction of lane 2 is the conventional system without any materials for optimizing, which is as a positive control; the materials for optimizing contained in each 25µL reaction system are as below: 20µg titanium powder and 1200µg chromium powder (lane 3), 800µg titanium powder and 300µg chromium powder (lane 4), 20µg titanium powder and 300µg chromium powder (lane 5), 800µg titanium powder and 1200µg chromium powder (lane 6), 0.3µL colloidal gold and 800µg titanium powder (lane 7), 1.6µL colloidal gold and 20µg titanium powder (lane 8), 0.3µL colloidal gold and 20µg titanium powder (lane 9), and 1.6µL colloidal gold and 800µg titanium powder (lane 10). The reaction of lane 11 is the negative control.

The amplification results of PCR containing suitable amount of the mixture of materials for optimizing mentioned above show a single predominant band with the reduction of non-specific amplification product. However, the amplification results of the positive control show severe smear. Furthermore, each component in the mixture in a lower amount than used alone can achieve the optimizing effect. The effect of optimization is notable in this experiment.

## Claims

1. A method of increasing the specificity and product yield in a PCR amplification reaction, **characterized in that** the method comprises the step of adding any one of the following elemental materials into a PCR reaction solution to optimize PCR amplification, wherein said elemental material is selected from a group consisting of elemental gold, elemental titanium, elemental nickel, elemental bismuth, elemental stibium, elemental selenium, and elemental chromium or mixtures thereof.

2. The method according to claim 1, **characterized in that** the said method comprises the following steps:
(1) preparing one of the elemental materials or the mixtures thereof;
(3) adding the elemental material or the mixtures from step (1) into a PCR reaction solution; and
(5) removing the elemental material or the mixtures from the PCR reaction solution to obtain the PCR products.

3. The method according to claim 1 or 2, **characterized in that** the said method comprises the following steps:
(1) preparing one of the elemental materials or the mixtures thereof;
(2) sterilizing the elemental material or the mixtures from step (1);
(3) adding the elemental material or the mixtures from step (2) into a PCR reaction solution;
(4) checking the PCR products by agarose gel electrophoresis; and
(5) removing the elemental material or the mixtures from the PCR reaction solution to obtain the PCR products.

4. The method according to any one of claims 1 to 3, **characterized in that** the elemental material is in the form of filament, sheet, particle, powder, colloidal or any other irregular forms.

5. The method according to any one of claims 1 to 4, **characterized in that** the step (1) is implemented by the followings: the material in filament, sheet, or any other irregular forms is washed sequentially in sterile water and anhydrous ethanol for multiple times, and then air dried for later use; or the material in particle or powder is washed sequentially in sterile water and anhydrous ethanol, and then dried in oven for later use.

6. The method according to any one of claims 1 to 5, **characterized in that** the amount of the elemental material added in step (3) is as the follows: in the final 25µL PCR reaction solution, the amount of 200-300 mesh titanium powder is ranged from 20µg to 1000µg, and the amount of other powder material is ranged from 50µg to 5000µg; or in the final 25µL PCR reaction solution, the amount of colloidal gold is ranged from 0.06µL to 6.0µL, and the amount of other colloidal material is ranged from 0.5µL to 4µL.

7. The method according to claim 6, **characterized in that** the amount of 200-300 mesh titanium powder is ranged from 50µg to 800µg.

8. The method according to claim 6, **characterized in that** the amount of 200 mesh chromium powder is ranged from 450µg to 1500µg.

9. The method according to claim 8, **characterized in that** the amount of 200 mesh chromium powder is ranged from 600µg to 1200 µg.

10. The method according to claim 6, **characterized in that** the amount of gold powder is equal to or more than 2mg.

11. The method according to claim 6, **characterized in that** the amount of 5nm size of colloidal gold with 0.01% HAuCl₄ concentration is ranged from 0.06µL to 0.5µL; the amount of 10nm size of colloidal gold with 0.01% HAuCl₄ concentration is ranged from 0.5µL to 5.0µL; and the amount of 20nm size of colloidal gold with 0.01% HAuCl₄ concentration is ranged from 0.8µL to 6.0µL.

12. The method according to claim 11, **characterized in that** the amount of 5nm size of colloidal gold with 0.01% HAuCl₄ concentration is ranged from 0.08µL to 0.16µL; the amount of 10nm size of colloidal gold with 0.01% HAuCl₄ concentration is ranged from 0.8µL to 1.6µL; and the amount of 20nm size of colloidal gold with 0.01% HAuCl₄ concentration is ranged from 0.8µL to 3.5µL.

13. The method according to any one of claims 1 to 5, **characterized in that** the amount of the mixture added in step (3) is as the follows: the mixture can be formed in any combination of the elemental materials and the amount of each material in the mixture is varied with the sort of the material and its form; the total amount of a mixture in powder form is ranged from 50µg to 5000µg; the total amount of a mixture in colloidal form is ranged from 0.06µL to 6.0µL; and for a mixture of powder and colloidal materials, the total amount of powder materials is ranged from 20µg to 5000µg and the total amount of colloidal materials is ranged from 0.02µL to 6.0µL.

14. The method according to any one of claims 1 to 13, **characterized in that** in step (5), the material is removed from the PCR reaction solution by the following methods: for the material in the form of filament, sheet, or any other irregular forms, the solution is directly absorbed out to be separated from the material; for the material in the form of powder or particle, the PCR reaction solution is centrifuged at a low speed, and then the supernatant is absorbed out to be separated from the material; for colloidal material, the PCR reaction solution is frozen overnight at -20°C, and is centrifuged at a high speed after thawing, and then the supernatant is absorbed out to be separated from the material.

## Patentansprüche

1. Verfahren zum Erhöhen der Spezifität und Produktausbeute in einer PCR-Amplifikationsreaktion, **dadurch gekennzeichnet, dass** das Verfahren den Schritt des Zugebens eines der folgenden elementaren Werkstoffe in eine PCR-Reaktionslösung zum Optimieren der PCR-Amplifikation umfasst, wobei der elementare Werkstoff aus einer aus elementarem Gold, elementarem Titan, elementarem Nickel, elementarem Bismuth, elementarem Stibium, elementarem Selen und elementarem Chrom oder Mischungen davon bestehenden Gruppe ausgewählt ist.

2. Verfahren nach Anspruch 1, **dadurch gekennzeichnet, dass** das Verfahren folgende Schritte umfasst:
(1) Präparieren eines der elementaren Werkstoffe oder der Mischungen davon;
(3) Zugeben des elementaren Werkstoffs oder der Mischungen aus Schritt (1) in eine PCR-Reaktionslösung; und
(5) Entfernen des elementaren Werkstoffs oder der Mischungen aus der PCR-Reaktionslösung, um die PCR-Produkte zu erhalten.

3. Verfahren nach Anspruch 1 oder 2, **dadurch gekennzeichnet, dass** das Verfahren folgende Schritte umfasst:
(1) Präparieren eines der elementaren Werkstoffe oder der Mischungen davon;
(2) Sterilisieren des elementaren Werkstoffs oder der Mischungen aus Schritt (1);
(3) Zugeben des elementaren Werkstoffs oder der Mischungen aus Schritt (2) in eine PCR-Reaktionslösung;
(4) Überprüfen der PCR-Produkte mittels einer Agarose-Gelelektrophorese; und
(5) Entfernen des elementaren Werkstoffs oder der Mischungen aus der PCR-Reaktionslösung, um die PCR-Produkte zu erhalten.

4. Verfahren nach einem der Ansprüche 1 bis 3, **dadurch gekennzeichnet, dass** der elementare Werkstoff eine Filament-, Blech-, Partikel-, Pulver-, kolloidale Form oder beliebige andere irreguläre Formen hat.

5. Verfahren nach einem der Ansprüche 1 bis 4, **dadurch gekennzeichnet, dass** der Schritt (1) wie folgt ausgeführt wird: Der Werkstoff in Filament-, Blech- oder beliebigen anderen Formen wird mehrmals aufeinanderfolgend in Sterilwasser und wasserfreiem Ethanol gewaschen und danach zur späteren Verwendung an der Luft getrocknet; oder der Werkstoff in Partikel- oder Pulverform wird aufeinanderfolgend in Sterilwasser und wasserfreiem Ethanol gewaschen und danach zur späteren Verwendung im Trockenschrank getrocknet.

6. Verfahren nach einem der Ansprüche 1 bis 5, **dadurch gekennzeichnet, dass** auf die Menge des in Schritt (3) zugegebenen elementaren Werkstoffs Folgendes zutrifft: In der abschließenden 25µL-PCR-Reaktionslösung liegt die Menge von Titanpulver mit 200-300 Mesh im Bereich von 20µg bis 1000µg und liegt die Menge von anderem pulverförmigen Werkstoff im Bereich von 50µg bis 5000µg; oder in der abschließenden 25µL-PCR-Reaktionslösung liegt die Menge von kolloidalem Gold im Bereich von 0,06µL bis 6,0µL und liegt die Menge von anderem kolloidalen Werkstoff im Bereich von 0,5µL bis 4µL.

7. Verfahren nach Anspruch 6, **dadurch gekennzeichnet, dass** die Menge von Titanpulver mit 200-300 Mesh im Bereich von 50µg bis 800µg liegt.

8. Verfahren nach Anspruch 6, **dadurch gekennzeichnet, dass** die Menge von Chrompulver mit 200 Mesh im Bereich von 450µg bis 1500µg liegt.

9. Verfahren nach Anspruch 8, **dadurch gekennzeichnet, dass** die Menge von Chrompulver mit 200 Mesh im Bereich von 600µg bis 1200µg liegt.

10. Verfahren nach Anspruch 6, **dadurch gekennzeichnet, dass** die Menge von Goldpulver gleich oder größer als 2mg ist.

11. Verfahren nach Anspruch 6, **dadurch gekennzeichnet, dass** die Menge von kolloidalem Gold in einer Größe von 5nm mit einer HAuCl₄-Konzentration von 0,01 % im Bereich von 0,06µL bis 0,5µL liegt; die Menge von kolloidalem Gold in einer Größe von 10nm mit einer HAuCl₄-Konzentration von 0,01 % im Bereich von 0,5µL bis 5,0µL liegt; und die Menge von kolloidalem Gold in einer Größe von 20nm mit einer HAuCl₄-Konzentration von 0,01 % im Bereich von 0,8µL bis 6,0µL liegt.

12. Verfahren nach Anspruch 11, **dadurch gekennzeichnet, dass** die Menge von kolloidalem Gold in einer Größe von 5nm mit einer HAuCl₄-Konzentration von 0,01 % im Bereich von 0,08µL bis 0,16µL liegt; die Menge von kolloidalem Gold in einer Größe von 10nm mit einer HAuCl₄-Konzentration von 0,01 % im Bereich von 0,8µL bis 1,6µL liegt; und die Menge von kolloidalem Gold in einer Größe von 20nm mit einer HAuCl₄-Konzentration von 0,01% im Bereich von 0,8µL bis 3,5µL liegt.

13. Verfahren nach einem der Ansprüche 1 bis 5, **dadurch gekennzeichnet, dass** auf die Menge der in Schritt (3) zugegebenen Menge Folgendes zutrifft: Die Mischung kann in einer beliebigen Kombination der elementaren Werkstoffe gebildet werden, und die Menge jedes Werkstoffs in der Mischung ist je nach der Art des Werkstoffs und dessen Form unterschiedlich; die Gesamtmenge einer Mischung in Pulverform liegt im Bereich von 50µg bis 5000µg; die Gesamtmenge einer Mischung in kolloidaler Form liegt im Bereich von 0,06µL bis 6,0µL; und für eine Mischung von pulverförmigen und kolloidalen Werkstoffen liegt die Gesamtmenge von pulverförmigen Werkstoffen im Bereich von 20µg bis 5000µg und liegt die Gesamtmenge von kolloidalen Werkstoffen im Bereich von 0,02µL bis 6,0µL.

14. Verfahren nach einem der Ansprüche 1 bis 13, **dadurch gekennzeichnet, dass** in Schritt (5) der Werkstoff mittels der folgenden Verfahren aus der PCR-Reaktionslösung entfernt wird:
Für den Werkstoff in Filament-, Blech- oder beliebigen anderen irregulären Formen wird die Lösung direkt heraus absorbiert, um vom Werkstoff getrennt zu werden; für den Werkstoff in Pulver- oder Partikelform wird die PCR-Reaktionslösung bei geringer Geschwindigkeit zentrifugiert, und danach wird der Überstand heraus absorbiert, um vom Werkstoff getrennt zu werden; für kolloidalen Werkstoff wird die PCR-Reaktionslösung über Nacht bei -20°C tiefgefroren und wird nach dem Auftauen bei hoher Geschwindigkeit zentrifugiert, und danach wird der Überstand heraus absorbiert, um vom Werkstoff getrennt zu werden.

## Revendications

1. Une méthode visant à augmenter la spécificité et le rendement d'un produit dans une réaction d'amplification en chaîne par polymérase (PCR), **se caractérisant par le fait que** la méthode suppose l'ajout de l'un quelconque des matériaux élémentaires suivants dans une solution de réaction PCR afin d'optimiser l'amplification PCR, dans laquelle ledit matériau élémentaire est sélectionné parmi les suivants : or élémentaire, titane élémentaire, nickel élémentaire, bismuth élémentaire, antimoine élémentaire, sélénium élémentaire et chrome élémentaire ou des mélanges de ces matériaux.

2. La méthode de la revendication 1, **se caractérisant par le fait que** la méthode comprend les opérations suivantes :
(1) préparation d'un des matériaux élémentaires ou des mélanges de matériaux élémentaires ;
(3) ajout du matériau élémentaire ou des mélanges résultant de l'opération (1) dans une solution de réaction PCR ; et
(5) retrait du matériau élémentaire ou des mélanges de la solution de réaction PCR afin d'obtenir les produits PCR.

3. La méthode de la revendication 1 ou 2, **se caractérisant par le fait que** la méthode comprend les opérations suivantes :
(1) préparation d'un des matériaux élémentaires ou des mélanges de matériaux élémentaires ;
(2) stérilisation du matériau élémentaire ou des mélanges préparés à l'étape (1) ;
(3) ajout du matériau élémentaire ou des mélanges stérilisés à l'étape (2) dans une solution de réaction PCR;
(4) contrôle des produits PCR par électrophorèse en gel d'agarose ; et
(5) retrait du matériau élémentaire ou des mélanges de la solution de réaction PCR pour obtenir les produits PCR.

4. La méthode de n'importe laquelle des revendications 1 à 3, **se caractérisant par le fait que** le matériau élémentaire se présente sous la forme de filament, de feuille, de particule ou de poudre ou sous forme colloïdale ou toute autre forme irrégulière.

5. La méthode de n'importe laquelle des revendications 1 à 4, **se caractérisant par le fait que** l'étape (1) a lieu comme suit : le matériau sous forme de filament, de feuille ou toute autre forme irrégulière est lavé de manière séquentielle dans de l'eau stérile et de l'éthanol anhydre plusieurs fois puis est séché à l'air en vue d'une utilisation ultérieure ; ou bien le matériau sous forme de particule ou de poudre est lavé de manière séquentielle dans de l'eau stérile et de l'éthanol anhydre puis est séché au four en vue d'une utilisation ultérieure.

6. La méthode de n'importe laquelle des revendications 1 to 5, **se caractérisant par le fait que** la quantité de matériau élémentaire ajoutée à l'étape (3) est la suivante : dans la solution de réaction PCR à 25 µL finale, la quantité de poudre de titane de 200-300 mesh va de 20 µg à 1 000 µg et la quantité des autres matériaux en poudre va de 50 µg à 5 000 µg ; ou dans la solution de réaction PCR à 25 µL finale, la quantité d'or colloïdal va de 0,06 µL à 6,0 µL et la quantité des autres matériaux colloïdaux va de 0,5 µL à 4 µL.

7. La méthode de la revendication 6, **se caractérisant par le fait que** la quantité de poudre de titane de 200-300 mesh va de 50 µg à 800 µg.

8. La méthode de la revendication 6, **se caractérisant par le fait que** la quantité de poudre de chrome de 200 mesh va de 450 µg à 1 500 µg.

9. La méthode de la revendication 8, **se caractérisant par le fait que** la quantité de poudre de chrome de 200 mesh va de 600 µg à 1 200 µg.

10. La méthode de la revendication 6, **se caractérisant par le fait que** la quantité de poudre d'or est égale ou supérieure à 2 mg.

11. La méthode de la revendication 6, **se caractérisant par le fait que** la quantité d'or colloïdal de 5 nm avec une concentration de 0,01 % HAuCl₄ va de 0,06µL à 0,5 µL ; la quantité d'or colloïdal de 10 nm avec une concentration de 0,01 % HAuC₄ va de 0,5 µL à 5,0 µL ; et la quantité d'or colloïdal de 20 nm avec une concentration de 0,01 % HAuCl₄ va de 0,8 µL à 6,0 µL.

12. La méthode de la revendication 11, **se caractérisant par le fait que** la quantité d'or colloïdal de 5 nm avec une concentration de 0,01 % HAuCl₄ va de 0,08 µL à 0,16 6 µL ; la quantité d'or colloïdal de 10 nm avec une concentration de 0,01 % HauCl₄ va de 0,8 µL à 1,6 µL; et la quantité d'or colloïdal de 20 nm avec une concentration de 0,01 % HAuCl₄ va de 0,8 µL à 3,5 µL.

13. La méthode de n'importe laquelle des revendications 1 à 5, **se caractérisant par le fait que** la quantité du mélange ajouté à l'étape (3) est la suivante : le mélange peut être formé de n'importe quelle combinaison des matériaux élémentaires et la quantité de chaque matériau du mélange varie en fonction du type de matériau et de sa forme ; la quantité totale d'un mélange sous forme de poudre va de 50 µg à 5 000 µg ; la quantité totale d'un mélange sous forme colloïdale va de 0,06 µL à 6,0 µL ; et pour un mélange de matériaux sous forme de poudre et sous forme colloïdale, la quantité totale de matériaux en poudre va de 20 µg à 5 000 µg et la quantité totale de matériaux colloïdaux va de 0,02 µL à 6,0 µL.

14. La méthode de n'importe laquelle des revendications 1 à 13, **se caractérisant par le fait que**, à l'étape (5), le matériau est retiré de la solution de réaction PCR par les méthodes suivantes : pour le matériau sous forme de filament, de feuille ou sous toute autre forme irrégulière, la solution est directement absorbée en vue d'être séparée du matériau ; pour le matériau sous forme de poudre ou de particule, la solution de réaction PCR est centrifugée à basse vitesse puis le surnageant est absorbé en vue d'être séparé du matériau ; pour le matériau colloïdal, la solution de réaction PCR est congelée pendant la nuit à -20°C et est centrifugée à haute vitesse une fois décongelée, et le surnageant est ensuite absorbé en vue d'être séparé du matériau.
